(19) Europäisches Patentamt
European Patent Office
Office européen des brevets

(11) Publication number: **0 034 835**
**B1**

(12) # EUROPEAN PATENT SPECIFICATION

(45) Date of publication of patent specification: **12.12.84**

(51) Int. Cl.$^3$: **C 07 J 9/00,** C 07 J 31/00

(21) Application number: **81101853.0**

(22) Date of filing: **10.07.79**

(60) Publication number of the earlier application in accordance with Art. 76 EPC: **0 007 277**

(54) **Cholesterol derivatives as intermediary products for the synthesis of immunologic adjuvants.**

(30) Priority: **11.07.78 US 923520**

(43) *Date of publication of application:*
**02.09.81 Bulletin 81/35**

(45) Publication of the grant of the patent:
**12.12.84 Bulletin 84/50**

(84) Designated Contracting States:
**AT BE CH DE FR GB IT LU NL SE**

(56) References cited:
**AUSTRALIAN JOURNAL OF CHEMISTRY no. 24 (1), January 1971, pages 143-151 Melbourne, AU M.S. AHMAD et al.: "Lithium Aluminium Hydride-Aluminium Chloride Reduction of Steroidal Cyclic Acetals"**

**The file contains technical information submitted after the application was filed and not included in this specification**

(73) Proprietor: **MERCK & CO. INC.**
**126, East Lincoln Avenue P.O. Box 2000**
**Rahway New Jersey 07065 (US)**

(72) Inventor: **Ponpirom, Mitree Michael**
**431 Brookview Court**
**Branchburg New Jersey 09976 (US)**
Inventor: **Chabala, John**
**530 Summit Avenue**
**Westfield New Jersey 07090 (US)**
Inventor: **Shen, Tsung-Ying**
**935 Minisink Way**
**Westfield New Jersey 07090 (US)**

(74) Representative: **Corre, Jacques Denis Paul et al**
**Cabinet Regimbeau 26, Avenue Kléber**
**F-75116 Paris (FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 034 835 B1

**Description**

Background of the invention

The present invention relates to intermediates of the formulae II, III and IV for the preparation of an immunologic adjuvant. The glycolipid immunologic adjuvant and improved vaccine formulations containing a said glycolipid immunologic adjuvant are described and claimed in EP—A 0 007 277.

Detailed description

The glycolipid compounds of EP—A—0 007 277 which are useful as immunologic adjuvants are prepared with reference to Table I starting from cholesterol tosylate I. The latter may be condensed with hexanediol in an aprotic solvent such as, for example, dioxane or tetrahydrofuran (THF) to yield the corresponding 6-hydroxyhexyl derivative II. The latter may be converted to the 6-p-toluenesulfonyloxy derivative III using known methods, e.g. treatment with p-toluenesulfonic anhydride or a p-toluenesulfonyl halide. The latter may be converted to the corresponding 6-iodo derivative IV using known methods, e.g., by treatment with a metallic iodide, e.g. LiI or NaI. The protected compounds of formula VI a-c may be obtained by reacting IV with Va-c. This reaction takes place in an aprotic solvent, preferably a halogenated aprotic solvent, such as, for example, $CH_2Cl_2$, $CHCl_3$, $CCl_4$, acetonitrile or benzene, and in the presence of an acid acceptor such as, for example, triethylamine (TEA) or 1,5-diazabicyclo [5,4,0]undec-5-ene. The final products of formula VII a-c may be obtained by deprotecting the compounds of formula VI a-c. This may be accomplished using known methods by saponification in the presence of a base such as, for example, $NaOCH_3$, $NH_3$, or an anionic ion exchange resin.

TABLE 1

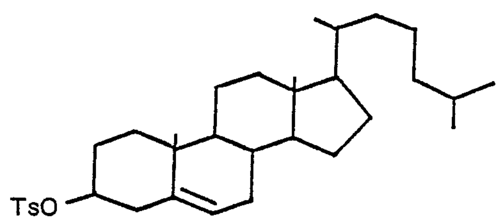

I

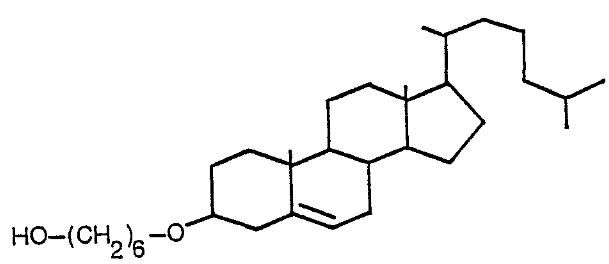

II

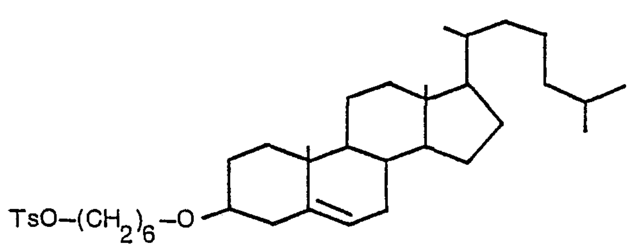

III

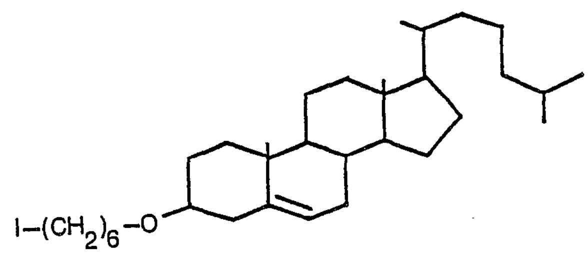

IV

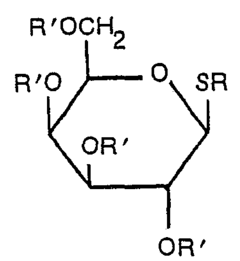

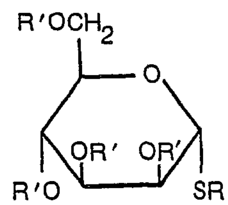

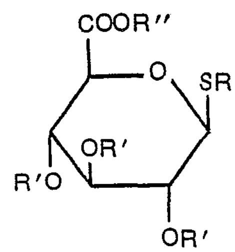

| | | | | | |
|---|---|---|---|---|---|
| *V*a | R = H, R′ = Ac | *V*b | R = H, R′ = Ac | *V*c | R = H, R′ = Ac, R″ = $CH_3$ |
| *VI*a | R = X, R′ = Ac | *VI*b | R = X, R′ = Ac | *VI*c | R = X, R′ = Ac, R″ = $CH_3$ |
| *VII*a | R = X, R′ = H | *VII*b | R = X, R′ = H | *VII*c | R = X, R′ = R″ = H |

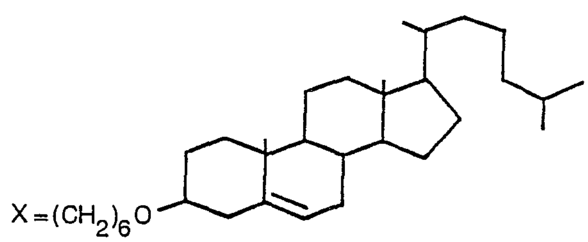

The final product of formula VIId may be obtained with reference to Table 2 starting from 1—1,2,3,4-tetra-*O*-acetyl-6-*O*-methylsulfonyl-D-mannose VIII and converting the latter to the corresponding $\alpha$-glycosyl bromide IX by bromination using known methods such as, for example, treatment with HBr in glacial acetic acid. The latter may be converted to the corresponding isothioronium bromide X using known methods such as, for example, treatment with thiourea in a polar solvent such as, for example, acetone, methylethyl ketone (MEK) or dimethylformamide (DMF) at a temperature about equal to the boiling point of the solvent. The isothiouronium bromide X may be converted to the corresponding thiol XI using known methods such as, for example, treatment with $K_2S_2O_5$ in an aprotic solvent, preferably a halogenated solvent, such as, for example, $CHCl_3$ or $CH_2Cl_2$ at a temperature about equal to the boiling point of the solvent. The thiol in turn may be coupled with IV using the conditions previously described to yield the compound of formula XII. The latter may be converted to the azide of formula *XIII* using known methods for example, by treatment with $NaN_3$ in a polar solvent such as, for example DMF, acetone or MEK. The protected compound of formula *XIII* may be deblocked using the conditions previously described to prepare the compound of formula *XIV* which then may be converted to the final amino product of formula *VII*d using known methods such as, for example, by treatment with $H_2S$.

TABLE 2

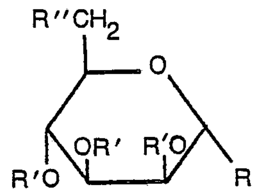

| | |
|---|---|
| *VII*d | $R = SX$, $R' = H$, $R'' = NH_2$ |
| *VIII* | $R = OAc$, $R' = Ac$, $R'' = OMs$ |
| *IX* | $R = Br$, $R' = Ac$, $R'' = OMs$ |
| *X* | $R = SC(=NH_2^+)NH_2Br^-$, $R' = Ac$, $R'' = OMs$ |
| *XI* | $R = SH$, $R' = Ac$, $R'' = OMs$ |
| *XII* | $R = SX$, $R' = Ac$, $R'' = OMs$ |
| *XIII* | $R = SX$, $R' = Ac$, $R'' = N_3$ |
| *XIV* | $R = SX$, $R' = H$, $R'' = N_3$ |

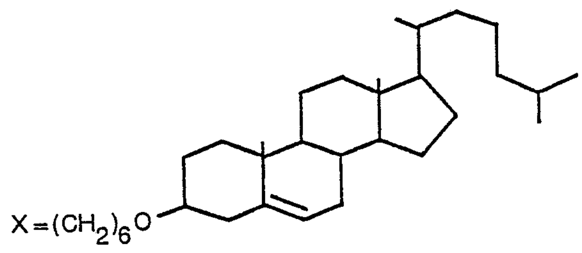

The adjuvants of EP—A—0 007 277 may be employed to potentiate the antibody response of antigenic materials. The term "antigen" and "antigenic material" which are used interchangeably herein include one or more non-viable immunogenic or desensitizing (anti-allergic) agents of bacterial, viral or other origin. The antigen component may consist of a dried powder, an aqueous solution, an aqueous suspension and the like, including mixtures of the same, containing a non-viable immunogenic or desensitizing agent or agents.

The aqueous phase may conveniently be comprised of the antigenic material in a parenterally acceptable liquid. For example, the aqueous phase may be in the form of a vaccine in which the antigen is dissolved in a balanced salt solution, physiological saline solution, phosphate buffered saline solution, tissue culture fluids or other media in which the organism may have been grown. The aqueous phase also may contain preservatives and/or substances conventionally incorporated in vaccine preparations. The adjuvant emulsions of the invention may be prepared employing techniques well known to the art.

The antigen may be in the form of purified or partially purified antigen derived from bacteria, viruses, rickettsia or their products, or extracts of bacteria, viruses, or rickettsia, or the antigen may be an allergen such as pollens, dusts, danders, or extracts of the same or the antigen may be in the form of a poison or a venom derived from poisonous insects or reptiles. In all cases the antigens will be in the form in which their toxic or virulent properties have been reduced or destroyed and which when introduced into a suitable host will either induce active immunity by the production therein of antibodies against the specific microorganisms, extract or products of microorganisms used in the preparation of the antigen, or, in the case of allergens, they will aid in alleviating the symptoms of the allergy due to the specific allergen. The antigens can be used either singly or in combination; for example, multiple bacterial antigens, multiple viral antigens, multiple mycoplasmal antigens, multiple rickettsial antigens, multiple bacterial or viral toxoids, multiple allergens or combinations of any of the foregoing products can be combined in the aqueous phase of the adjuvant composition of this invention. Antigens of particular importance are derived from bacteria such as *B. pertussis, Leptospira pomona* and *icterohaemorrhagiae, S., typhosa, S. paratyphi* A and B, C. *diphtheriae, C. tetani, C. botolinum, C. perfringens, C. feseri* and other gas gangrene bacteria, *B. anthracis, P, pestis, P. multocida, V. cholerae, Neisseria meningitidis, N, gonorrheae, Hemophilus influenzae, Treponema pollidum,* and the like; for viruses as polio virus (multiple types), adeno virus (multiple types), parainfluenza virus (multiple types), measles, mumps. respiratory syncytial virus, influenza (various types), shipping fever virus ($SF_4$), Western and Eastern equine encephalomyelitis, Japanese B. encephalomyelitis, Russion Spring Summer encephalomyelitis, hog cholera virus, Newcastle disease virus, fowl pox, rabies, feline and canine distemper and the like viruses, from rickettsiae as epidemic and

endemic typhus or other members of the spotted fever group, from various spider and snake venoms or any of the known allergens for example from ragweed, house dust pollen extracts, grass pollens and the like.

The following examples illustrate the present invention without, however, limiting the same thereto.

Example 1

Preparation of Cholest-5-en-3β-yl 6-hydroxyhexyl Ether (II)

Using the procedure of Davis, J. Chem. Soc. 178—181 (1962), cholesterol tosylate and 1,6-hexanediol were condensed in refluxing dioxane to provide 52% yield of the title ether as colorless plates from hexane, m.p. 79.5—81°C; $[\alpha]_D^{25}$ −28.1 ±0.5°C; IR 3500—3200 (OH), 1100 and 1080 cm$^{-1}$ (ether); NMR ($CDCL_3$): δ2.8—3.3 (br, 1H, H—1), 3.3—3.75 (2t, 4H), 5.4 (m, 1H).

*Anal.* Calc. for $C_{33}H_{58}O_2$: C, 81.42; H, 12.01. Found: C, 81.74; H, 11.78.

Example 2

Preparation of Cholest-5-en-3β-yl 6-iodohexyl Ether IV

The alcohol, prepared by the process of Example 1 (16.0 g, 32.9 mmol) in 650 ml. dry benzene was treated with 11.9 g (36.3 mmol) *p*-toluenesulfonic anhydride prepared by the process of L. Field and J. W. McFarland, *Org. Syn. Coll.*, Vol. IV (1963) 940—942, and 5.8 ml (36 mmol) collidine, and stirred 1 hour at room temperature with the exclusion of moisture. The mixture was filtered through Florisil and concentrated to 16.0 g (76%) waxy cholest-5-en-3β-yl 6-(*p*-tolylsulfonyloxy) hexyl ether (III), homogeneous on TLC (15% EtOAc in benzene on silica gel); IR 1189 and 1175 (sulfonate), 1092 and 1087 (ether) cm$^{-1}$; NMR ($CDCl_3$): δ2.43 (s, 3H), 2.7—3.3 (m, overlapping t, 3H), 3.90 (t, 2H), 5.3 (m, 1H), 7.24 and 7.70 (d of d, 4H). This tosylate (14.2 g, 22.2 mmol) and 7.0 g. (47 mmol) NaI were refluxed together for 4 hours in 120 ml acetone. Solvent was removed under reduced pressure. The residue was treated with 75 ml ether, filtered, and the collected salts washed well with ether. The filtrate was evaporated and the residual yellow oil boiled with 200 ml hexane. The solution was decanted, concentrated to 100 ml, and refrigerated 2 days, depositing 9.85 g white needles. A second crop afforded 2.90 g (total yield: 12.75 g, 97%), m.p. 103.5—104.5°C; $[a]_D^{25}$ −22.6 ± 0.5 (c = 1.02 $CHCl_3$); IR 1105 (ether) cm$^{-1}$.

*Anal.* Calc. for $C_{33}H_{57}IO$: C, 66.42; H, 9.63. Found: C, 66.32; H, 9.55.

Example 3

Preparation of Glycosyl thiols

2,3,4,6-Tetra-*O*-acetyl-1-thio-β-D-galactopyranose (Va) was prepared by the process set forth in M. Cerny, J. Stanek, and J. Pacak, *Monatsh., 94* (1963) 290—294 and 2,3,4,6-tetra-*O*-acetyl-1-thio-1-α-D-mannopyranose (*Vb*) was prepared using the procedure set forth in K. L. Matta, R. N. Girotra, and J. J. Barlow, *Carbohydrate Res., 43* (1975) 101—109.

Methyl 2,3,4-tri-*O*-acetyl-1-thio-β-D-glycopyranosuronate (*Vc*) was prepared from the bromide described in W. D. S. Bowering and T. E. Timell, *J. Am. Chem. Soc., 82* (1960) 2827—2830 according to the method set forth in M. Cerny, J. Stanek and J. Pacak, *Monatsh., 94,* 290—294 (1963), requiring the use of 2-butanone for the preparation of the intermediate isothiouronium bromide. Needles, m.p. 129.5—130°C; $[\alpha]_D^{25}$ −4.2 ± 0.5° (c 1.0 $CHCl_3$); IR 2550 (thiol), 1740 (acetate) cm$^{-1}$; NMR ($CDCl_3$): δ2.03 (s, 6H), 2.10 (s, 3H), 2.33 (d, J 10 Hz, 1H), 3.78 (s, 3H).

*Anal.* Calc. for $C_{13}H_{18}O_9S$: C, 44.57; H, 5.18; S, 9.15. Found: C, 44.41; H, 4.97; S, 8.94.

Example 4

Preparation of Protected Glycolipids

To a solution of 1 equivalent glycosyl thiol in $CH_2Cl_2$ (20 ml per g thiol) was added 1 equivalent cholest-5-en-3β-yl 6-iodohexyl ether (*IV*) and 1 equivalent triethylamine. After stirring under $N_2$ at room temperature overnight, the mixture was chromatographed on silica gel using gradient elution with 5—25% ethyl acetate in benzene to yield the following compounds:

6-(Cholest-5-ene-3β-yloxy)hexyl 2,3,4,6-tetra-*O*-acetyl-1-thio-α-D-mannopyranoside (VIb)

55%, needles, m.p. 103—105°C (for hexane); $[\alpha]_D^{25}$ +34.3 ± 0.5° (c 1.00 $CHCl_3$); IR 1730 cm$^{-1}$; (acetate).

*Anal.* Calc. for $C_{47}H_{75}O_{10}S$: C, 67.75; H, 9.19; S, 3.85. Found: C, 67.92; H, 9.19; S, 3.85.

6-(Cholest-5-en-3β-yloxy)hexyl 2,3,4,6-tetra-*O*-acetyl 1-thio-β-D-galactopyranoside (VIa)

93% wax; $[\alpha]_D^{25}$ −23.0 ± 0.5° (c 1.06 $CHCl_3$); IR 1740 cm$^{-1}$.

*Anal.* Calc. for $C_{47}H_{76}O_{10}S$: C, 67.75; H, 9.19; S, 3.85. Found: C, 67.89; H, 8.89; S, 3.77.

Methyl [6-(cholest-5-en-3β-yloxy)hexyl 2,3,4-tri-O-acetyl-1-thio-β-D-glucopyranoside]uronate (VIc)

48%, needles (from 15% benzene in hexane), m.p. 144.5—145°C; $[\alpha]_D^{25}$ −42.9 ± 0.5° (c 1.01 $CHCl_3$); IR 1735 (ester) cm$^{-1}$.

*Anal.* Calc. for $C_{47}H_{74}O_{10}S$: C, 67.45; H, 9.11; S, 3.91. Found: C, 67.68; H, 9.44; S, 4.20.

Example 5

Process for deblocking of neutral glycolipids

A solution of 1 equivalent glycolipid in 1:1 ethanol-THF (33 ml per g substrate) was treated with 2.5—3 fold excess Bio-Rad AG 1—X2 $OH^-$ ion-exchange resin suspended in ethanol (16 ml per g substrate) and stirred 45 minutes at room temperature. The resin was filtered off and washed with warm THF (3 x 16 ml per g substrate), and the combined filtrates evaporated to give the following compounds:

6-(Cholest-5-en-3β-yloxy)hexyl 1-thio-α-*D*-mannopyranoside (VIIb)

91% colorless needles (from THF), d.s.c. endothermic transitions; 64—65, 81—82, and 226—227°C; $[\alpha]_D^{25}$ +77.9 ± 0.9° (c 1.11 THF); IR 3600—3100 (OH) $cm^{-1}$; (s), 1105 (s), 1070 (s) $cm^{-1}$; MS: 665 ($M^+$), 501, 368.

*Anal.* Calc. for $C_{39}H_{68}O_6S$: C, 70.44; H, 10.31; S, 4.82. Found: C, 70.20; H, 10.22; S, 4.80.

6-(Cholest-5-en-3β-yloxy)hexyl 1-thio-β-*D*-galactopyranoside (VIIa)

66% pale tan powder, m.p. 104—106°C (to liquid crystal) and 219—221°C (to isotropic liquid); IR 3600—3100 (OH) $cm^{-1}$.

*Anal.* Calc. for $C_{39}H_{68}O_6S$: C, 70.44; H, 10.31; S, 4.82. Found: C, 70.16; H, 10.03; S, 4.79.

[6-(Cholest-5-en-3β-yloxy)hexyl 1-thio-β-*D*-glucopyranosid]uronic acid (VIIc)

A solution of 30.2 mg (39.1 μmol) esterified glycolipid *VIc* in 2.0 ml 1:1 methanol-THF containing 20 μl $H_2O$ was treated with 7.4 mg. (137 μmol) $NaOCH_3$ and stirred 3 hours at 25°C. The mixture was treated with 60 μl 2.5 *N* HCl (10% excess), evaporated, taken up in THF, and filtered. Evaporation of THF left 13.7 mg (52%) white powder, m.p. 104—107°C; IR 3600—2800 (RCOOH), 1745 and 1728 (RCOOH) $cm^{-1}$; field desorption MS: 701 ($M^+$, $Na^+$ salt). A 1 mg sample was per-*O*-trimethylsilylated with bis(trimethylsilyl)trifluoroacetamide at 25°C in DMF and analyzed by MS: 967 ($M^+$), 942, 874, 859, 501, 465. High resolution MS: Calc. for $C_{33}H_{57}OS^+$: 501.4126; Found: 501,4126; Calc. for $C_{18}H_{41}O_6Si_4^+$: 465.1982 Found: 465.1965.

*Anal.* Calc. for $C_{39}H_{66}O_7S$: C, 68.99, H, 9.79; S, 4.72. Found: C, 69.48; H, 9.59; S, 4.65.

Example 6

Preparation of 2,3,4-tri-O-acetyl-6-O-methylsulfonyl-α-D-mannopyranosyl Bromide

An ice-cold solution of 14.9 g (34.9 mmol) 1,2,3,4-tetra-*O*-acetyl-6-*O*-methylsulfonyl-β-D-mannopyranose prepared by the process set forth in J. Fernandez-Bolanos and R. G. Fernandez-Bolanos, *An. Quim., 65* (1969) 1163—1164; *Chem. Abstr., 72* (1970) 133105w in 60 ml dry $CH_2Cl_2$ was treated with 21 ml 30—32% HBr in glacial AcOH, and kept 2.5 hours at 25°C. The mixture was poured into 400 ml stirred ice water, separated, and the aqueous phase washed with three 20 ml aliquots of $CH_2Cl_2$. The combined organic layers were washed with water and saturated $NaHCO_3$, dried with $Na_2SO_4$, evaporated, triturated with petroleum ether (bp 30—60°C), filtered, and air dried to leave 14.6 g (93%) of the solid α-glycosyl bromide IX, m.p. 167.5—168.5°C (dec); $[\alpha]_D^{25}$ + 120.0 ± 0.5° (c 1.01 $CHCl_3$); IR 1740 and 1725 (acetate) $cm^{-1}$.

*Anal.* Calc. for $C_{13}H_{19}BrO_{10}S$: C, 34.91; H, 4.28; Br, 17.87; S, 7.17. Found: C, 35.04; H, 4.18; Br, 17.61; S, 7.27.

Example 7

6-(Cholest-5-en-3β-yloxy)hexyl 2,3,4-tri-*O*-acetyl-6-azido-6-deoxy-1-thio-α-D-mannopyranoside (XIII)

The mannosyl bromide prepared by the process set forth in Example 6 was converted *via* the isothiouronium bromide *X* [85%, white powder, m.p. 87—90°C (dec); IR 3600—3100, 1740, 1640 $cm^{-1}$] to the corresponding thiol *XI* [89% colorless glass, IR 2560, 1750—1730 $cm^{-1}$] according to the process set forth in M. Cerny, J. Stanek and J. Pacak, *Monatsh. 94* 290—294 (1963). The thiol *XI* was coupled with *IV* as in Example 4 to provide 61% 6-(cholest-5-en-3β-yloxy)-hexyl 2,3,4-tri-*O*-acetyl-6-*D*-methylsulfonyl-1-thio-α-*D*-mannopyranoside (XII), a white glass, IR 1740 $cm^{-1}$. Finally, 1.14 g (1.32 mmol) of the preceding mesylate, 0.49 g (7.5 mmol) $NaN_3$, and 40 ml dry DMF were stirred together at 70—75°C under $N_2$ for 4.5 hours. After evaporating the solvent, the residue was dissolved in 20 ml ether, washed with three 10 ml portions of $H_2O$, dried with $MgSO_4$, and evaporated to leave 1.05 g (97%) title azide *XIII* as a colorless glass, homogenous on TLC (12% EtOAc in benzene on silica gel), $[\alpha]_D^{25}$ +12.6 ± 0.5° (c 1.02 $CHCl_3$); IR 2095 (azide), and 1755 (acetate) $cm^{-1}$. A 0.54 g sample was further purified by column chromatography (silica gel, 12% EtOAc in benzene), providing 0.417 g (75% yield), colorless glass which solidified on prolonged standing, m.p. 68—70°C.

*Anal.* Calc. for $C_{45}H_{73}N_3O_8S$: C, 66.22: H, 9.02; N, 5.15; S, 3.93. Found: C, 66.53; H, 8.98; N, 5.04; S, 3.98.

Example 8

Preparation of 6-(Cholest-5-en-3$\beta$-yloxy)hexyl 6-azido-6-deoxy-1-thio-$\alpha$-D-mannopyranoside (XIV)

Azido triacetate *XIII* was deblocked by the process set forth in Example 5 to provide 62% of the title azido triol *XIV* as a glass, IR 3600—3100 (OH), 2095 (azide) $cm^{-1}$.

*Anal.* Calc. for $C_{39}H_{67}N_3O_5S$: C, 67.88; H, 9.79; S, 4.65. Found C, 67.80; H, 9.55; S, 4.54.

Example 9

Preparation of 6-(Cholest-5-en-3$\beta$-yloxy)hexyl 6-amino-6-deoxy-1-thio-$\alpha$-D-mannopyranoside (VIId)

A solution of 0.163 g (0.236 mmol) of azido triol prepared by the process set forth in Example 8 in 4.0 ml $CHCl_3$ containing 3.0 ml triethylamine was treated with dry gaseous $H_2S$ at room temperature for 4.5 hours. The volatile components were removed by rotary evaporation and the product isolated by preparative TLC (silica gel, 7:2:1 $CHCl_3/CH_3OH$/conc. aqueous $NH_3$) to yield 65.0 mg (41%) white powder, m.p. indef.; IR 3650 ($NH_2$), 3600—3100 (OH) $cm^{-1}$; MS: 635 ($M^+ - CH_2{=}NH^+$), 503, 470, 386, 368, 353. A 1 mg sample was per-*O*-trimethylsilylated with bis(trimethylsilyl)trifluoroacetamide in DMF at 65—70°C for 15 minutes, MS: 1024 ($M^+$), 1009, 951 ($M^+ - TMS$), 936, 921, 850 $[M^+ - N(Si[CH_3]_3)_2$, 523, 501, 368, 174 (base peak). A high resolution MS of the per-*O*-trimethylsilylated saccharide fragment was taken.

*Anal.* Calc. for $C_{21}H_{52}NO_4Si_5^+$: 522.2744. Found 522.2721.

Example 10

An aqueous suspension of the final product of formula VIIa in PBS is sterile filtered and added in levels of 0.005 mg and 0.05 mg of 2 samples of bivalent whole influenza vaccine (A Victoria and B Hong Kong strains). Similar adjuvant vaccine preparations are prepared using the final products of formula VIIb, VIIc and VIId.

Example 11

The procedure of Example 10 is repeated using sub-unit A victoria influenza vaccine.

**Claim for the contracting States: BE CH DE FR GB IT LU NL SE**

1. A compound of the formula

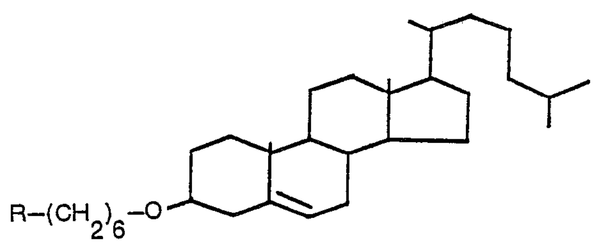

wherein R is OH, p-toluenesulfonyloxy, I.

**Claims for the contracting State: AT**

1. A process for the preparation of a compound of the formula

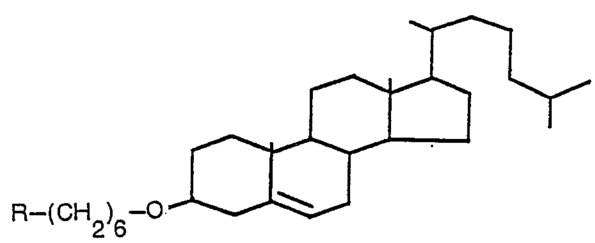

wherein R is OH, p-toluenesulfonyloxy, I, characterized by the fact that cholesterol tosylate and 1,6-hexanediol are condensed to obtain cholest-5-en-3$\beta$-yl 6-hydroxyhexyl ether and if necessary the obtained compound is reacted with p-toluenesulfonic anhydride or a p-toluenesulfonyl halide to obtain cholest-5-en-3$\beta$-yl 6-(p-tolylsulfonyloxy)hexyl ether, and if necessary the obtained compound is reacted with a metallic iodide to obtain cholest-5-en-3$\beta$-yl 6-iodohexyl ether.

2. A process according to claim 1, characterized by the fact that said metallic iodide is LiI or NaI.

**Patentanspruch fü die Vertragsstaaten: BE CH DE FR GB IT LU NL SE**

1. Verbindung der Formel

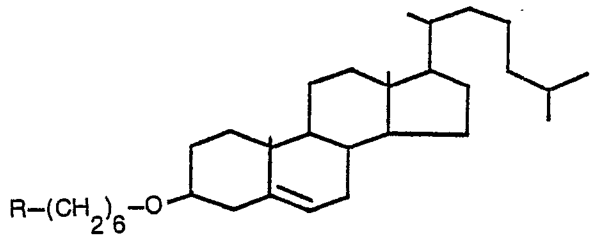

worin R OH, p-Toluolsulfonyloxy, I ist.

**Patentansprüche für den Vertragsstaat: AT**

1. Ein Verfahren zur Herstellung einer Verbindung der Formel

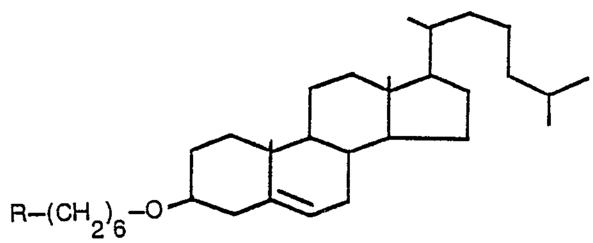

worin R OH, p-Toluolsulfonyloxy oder I ist, dadurch gekennzeichnet, daß man Cholesterintosylat und 1,6-Hexandiol zur Gewinnung von Cholest-5-en-3β-yl-6-hydroxyhexylether kondensiert, erforderlichenfalls die erhaltene Verbindung mit p-Toluolsulfonsäureanhydrid oder einem p-Toluolsulfonylhalogenid zur Gewinnung von Cholest-5-en-3β-yl-6-(p-tolylsulfonyloxy)hexylether umsetzt und erforderlichenfalls die erhaltene Verbindung mit einem Metalljodid zur Gewinnung von Cholest-5-en-3β-yl-6-jodhexyl-ether umsetzt.

2. Ein Verfahren nach Anspruch 1, dadurch gekennzeichnet, daß das genannte Metalljodid LiI oder NaI ist.

**Revendication pour les Etats contractants: BE CH DE FR GB IT LU NL SE**

1. Un composé de formule:

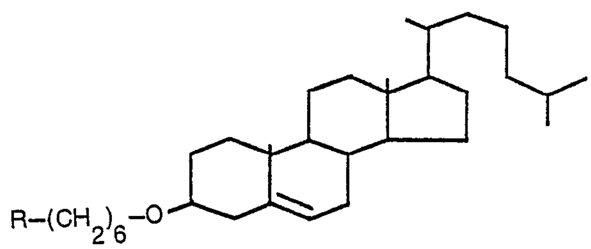

dans laquelle R est OH, un p-toluènesulfonyloxy ou I.

**Revendications pour l'Etat contractant: AT**

1. Procédé pour la préparation d'un composé de formula:

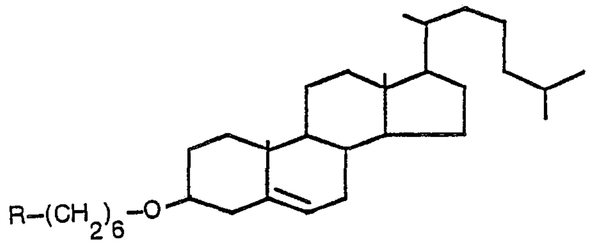

dans laquelle R représente OH, p-toluènesulfonyloxy, I, caractérisé en ce que le tosylate de cholestérol et le 1,6-hexanediol sont condensés pour obtenir l'éther cholest-5-en-3β-yl 6-hydroxyhexyl et si nécessaire le composé obtenu est mis à réagir avec l'anhydride p-toluènesulfonique ou un halogénure p-toluènesulfonyl pour obtenir l'éther cholest-5-en-3β-yl 6-(p-tolylsulfonyloxy)hexyl, et si nécessaire le composé obtenu est mis à réagir avec l'iodure métallique pour obtenir l'éther cholest-5-en-3β-yl 6-iodohexyl.

2. Procédé selon la revendication 1, caractérisé en ce que ledit iodure métallique est LiI ou NaI.